# EUROPÄISCHE PATENTSCHRIFT

(11) **EP 4 055 617 B1**
(45) Veröffentlichungstag und Bekanntmachung des Hinweises auf die Patenterteilung: **31.05.2023**
(21) Anmeldenummer: 20737023.0
(22) Anmeldetag: 09.07.2020
(51) Int. Cl.: H01B 1/02, C07F 15/00, A61K 8/23, C07F 9/66, C07F 1/00

(54) **ZUBEREITUNGEN VON PLATINKOMPLEXEN**
PREPARATION OF PLATINUM COMPLEXES
PRÉPARATIONS DES COMPLEXES DE PLATINE

(30) Priorität: 06.11.2019 EP 19207342
(43) Veröffentlichungstag der Anmeldung: 14.09.2022
(73) Patentinhaber: Heraeus Deutschland GmbH & Co. KG, 63450 Hanau (DE)
(72) Erfinder: SIEVI, Robert, 63450 Hanau (DE); GOCK, Michael, 63450 Hanau (DE); WALTER, Richard, 63450 Hanau (DE); ULLAND, Holger, 47877 Willich (DE)
(74) Vertreter: Heraeus IP
(86) Internationale Anmeldenummer: PCT/EP2020/069375
(87) Internationale Veröffentlichungsnummer: WO 2021/089203

(56) Entgegenhaltungen:
- WO-A1-92/10529
- WO-A1-2016/044732
- WO-A1-2017/035171
- JP-A- H1 018 036
- JP-A- H08 157 490

## Beschreibung

Die vorliegende Erfindung betrifft Zubereitungen von Platinkomplexen und die Verwendung der Zubereitungen zur Herstellung von Platinschichten auf Substraten.

WO90/07561 A1 offenbart Platinkomplexe der Formel LM[O(CO)R]₂, wobei L für einen stickstofffreien cyclischen Polyolefinliganden, bevorzugt Cyclooctadien (COD) oder Pentamethylcyclopentadien, und M für Platin oder Iridium steht, und wobei R Benzyl, Aryl oder Alkyl mit vier oder mehr Kohlenstoffatomen, besonders bevorzugt Phenyl bedeutet. Die Platinkomplexe dienen als Treibstoff additive.

Aufgabe der vorliegenden Erfindung war es, Zubereitungen zu finden, welche zur Herstellung von Platinschichten auf Substraten einschließlich temperaturempfindlichen Substraten verwendet werden können.

Die Aufgabe kann gelöst werden durch Bereitstellung einer Zubereitung enthaltend oder bestehend aus:
(A) 30 bis 90 Gew.-% (Gewichts-%) mindestens eines organischen Lösemittels,
(B) 10 bis 70 Gew.-% mindestens eines Platinkomplexes vom Typ [L1L2Pt[O(CO)R1]X]ₙ,
   wobei L1 und L2 gleiche oder verschiedene Monoolefinliganden oder zusammen eine als Diolefinligand fungierende Verbindung L1L2 bedeuten,
   wobei X ausgewählt ist unter Bromid, Chlorid, lodid und -O(CO)R2,
   wobei -O(CO)R1 und -O(CO)R2 gleiche oder verschiedene nichtaromatische C6-C18- oder bevorzugt C8-C18-Monocarbonsäurereste oder zusammen einen nichtaromatischen C8-C18-Dicarbonsäurerest -O(CO)R1 R2(CO)O- bedeuten,
   wobei es sich um einkernige Platinkomplexe mit n = 1 handelt oder wobei es sich im Falle der Anwesenheit von L1L2 und/oder von -O(CO)R1R2(CO)O- um mehrkernige Platinkomplexe mit ganzzahligem n > 1 handeln kann, und
(C) 0 bis 10 Gew.-% mindestens eines Additivs.

Bei einer bevorzugten Ausführungsform handelt es sich um Zubereitungen, wobei der 10 bis 70 Gew.-% ausmachende Bestandteil (B) aus mindestens einem Platinkomplex vom Typ [L1L2Pt[O(CO)R1]X]ₙ besteht, wobei L1 und L2 zusammen eine als Diolefinligand fungierende Verbindung L1L2 bedeuten,
wobei X ausgewählt ist unter Bromid, Chlorid, lodid und -O(CO)R2,
wobei -O(CO)R1 und -O(CO)R2 gleiche oder verschiedene nichtaromatische C6-C18- oder bevorzugt C8-18-Monocarbonsäurereste bedeuten, und
wobei es sich um einkernige Platinkomplexe mit n = 1 oder um mehrkernige Platinkomplexe mit ganzzahligem n > 1 handelt. L1L2 bedeutet hier eine als Diolefinligand fungierende Verbindung.

Im Falle mehrkerniger Platinkomplexe bedeutet die Zahl n im Allgemeinen eine ganze Zahl beispielsweise im Bereich von 2 bis 5. Anders ausgedrückt, ganzzahliges n > 1 liegt im Allgemeinen im Bereich von 2 bis 5; insbesondere ist n dann gleich 2 und es handelt sich um zweikernige Platinkomplexe. Insbesondere die Verbindung L1L2 respektive der Dicarbonsäurerest -O(CO)R1R2(CO)O- fungieren in den mehrkernigen Platinkomplexen als verbrückende Liganden. Auch X kann verbrückend wirken.

Das Platin liegt in den Platinkomplexen in der Oxidationsstufe +2 vor.

In der erfindungsgemäßen Zubereitung liegt der Bestandteil (B) gelöst in Bestandteil (A) vor. Bei Vorhandensein des optionalen Bestandteils (C) in der erfindungsgemäßen Zubereitung, so liegt auch dieser Bestandteil (C) bevorzugt gelöst in Bestandteil (A) vor. Mit anderen Worten, bei Fehlen des optionalen Bestandteils (C), handelt es sich bei der erfindungsgemäßen Zubereitung um eine organische Lösung, genauer gesagt um eine echte, d.h. nichtkolloidale organische Lösung; bei Vorhandensein des optionalen Bestandteils (C) in der bevorzugten Form, d.h. in im Bestandteil (A) gelöster Form, gilt das Gleiche.

Die erfindungsgemäße Zubereitung enthält 30 bis 90 Gew.-% mindestens eines organischen Lösemittels (A). Das oder die organischen Lösemittel können aus einer Vielzahl üblicher organischer Lösemittel ausgewählt sein, da die Platinkomplexe gut bis unbegrenzt in solchen organischen Lösemitteln löslich sind. Zweckmäßigerweise sind das oder die organischen Lösemittel unter den Verarbeitungsbedingungen der erfindungsgemäßen Zubereitung im Wesentlichen flüchtig, insbesondere gilt dies für das Stadium nach Applikation der erfindungsgemäßen Zubereitung auf ein Substrat. Im Allgemeinen liegen die Siedepunkte des oder der organischen Lösemittel im Bereich von 50 bis 200°C oder höher, beispielsweise 50 bis 300°C. Beispiele für organische Lösemittel (A) umfassen Aliphaten und Cycloaliphaten jeweils mit 6 bis 12 Kohlenstoffatomen; Halogenkohlenwasserstoffe wie Di-, Tri- und Tetrachlormethan; Aromaten; Araliphaten wie Toluol oder Xylol; Alkohole wie Ethanol, n-Propanol und Isopropanol; Ether; Glykolether wie Mono-C1-C4-alkylglykolether und Di-C1-C4-alkylglykolether, beispielsweise Ethylenglykolmono-C1-C4-alkylether, Ethylenglykoldi-C1-C4-alkylether, Diethylenglykolmono-C1-C4-alkylether, Diethylenglykoldi-C1-C4-alkylether, Propylenglykolmono-C1-C4-alkylether, Propylenglykoldi-C1-C4-alkylether, Dipropylenglykolmono-C1-C4-alkylether und Dipropylenglykoldi-C1-C4-alkylether; Ester mit 2 bis 12 Kohlenstoffatomen; und Ketone wie Aceton, Methylethylketon, Methylisobutylketon und Cyclohexanon. Araliphaten wie Toluol oder Xylol, Alkohole wie Ethanol, n-Propanol und Isopropanol und Glykolether wie Mono-C1-C4-alkylglykolether und Di-C1-C4-alkylglykolether, beispielsweise Ethylenglykolmono-C1-C4-alkylether, Ethylenglykoldi-C1-C4-alkylether, Diethylenglykolmono-C1-C4-alkylether, Diethylenglykoldi-C1-C4-alkylether, Propylenglykolmono-C1-C4-alkylether, Propylenglykoldi-C1-C4-alkylether, Dipropylenglykolmono-C1-C4-alkylether und Dipropylenglykoldi-C1-C4-alkylether sind dabei bevorzugt. Besonders bevorzugt besteht Bestandteil (A) respektive das mindestens eine organische Lösemittel (A) aus mindestens einem Alkohol, speziell mindestens einem der beispielhaft genannten Alkohole, und/oder aus mindestens einem Glykolether, speziell mindestens einem der beispielhaft genannten Glykolether. Insbesondere bevorzugt als Bestandteil (A) sind entsprechende Mischungen aus 30 bis 70 Gew.% Alkohol und dem zu 100 Gew.-% fehlenden Gewichtsanteil Glykolether.

Die erfindungsgemäße Zubereitung enthält 10 bis 70 Gew.-% mindestens eines Platinkomplexes vom Typ [L1L2Pt[O(CO)R1]X]ₙ (B). Der dem mindestens einen Platinkomplex entstammende Platingehalt der erfindungsgemäßen Zubereitung kann beispielsweise im Bereich von 2,5 bis 25 Gew.-% liegen.

In einer ersten Ausführungsform einkerniger Platinkomplexe vom Typ L1L2Pt[O(CO)R1]X handelt es sich bei L1 und L2 um gleiche oder verschiedene Monoolefinliganden; X bedeutet Bromid, Chlorid, lodid oder -O(CO)R2; und -O(CO)R1 und -O(CO)R2 bedeuten gleiche oder verschiedene nichtaromatische C6-C18- oder bevorzugt C8-C18-Monocarbonsäurereste, Phenylessigsäurereste bevorzugt jeweils ausgenommen.

In einer zweiten und zugleich bevorzugten Ausführungsform einkerniger Platinkomplexe vom Typ L1L2Pt[O(CO)R1]X handelt es sich bei L1 und L2 zusammen um eine am gleichen Platinzentralatom als Diolefinligand fungierende Verbindung L1L2; X bedeutet Bromid, Chlorid, lodid oder -O(CO)R2; und -O(CO)R1 und -O(CO)R2 bedeuten gleiche oder verschiedene nichtaromatische C6-C18- oder bevorzugt C8-C18-Monocarbonsäurereste, Phenylessigsäurereste bevorzugt jeweils ausgenommen.

In einer dritten Ausführungsform einkerniger Platinkomplexe vom Typ L1L2Pt[O(CO)R1]X handelt es sich bei L1 und L2 um gleiche oder verschiedene Monoolefinliganden; X bedeutet - O(CO)R2; -O(CO)R1 und -O(CO)R2 bedeuten zusammen einen nichtaromatischen als zweizähniger Ligand am gleichen Platinzentralatom fungierenden C8-C18-Dicarbonsäurerest - O(CO)R1R2(CO)O-.

In einer vierten Ausführungsform einkerniger Platinkomplexe vom Typ L1L2Pt[O(CO)R1]X handelt es sich bei L1 und L2 zusammen um eine am gleichen Platinzentralatom als Diolefinligand fungierende Verbindung L1L2; X bedeutet -O(CO)R2; und -O(CO)R1 und - O(CO)R2 bedeuten zusammen einen nichtaromatischen als zweizähniger Ligand am gleichen Platinzentralatom fungierenden C8-C18-Dicarbonsäurerest -O(CO)R1R2(CO)O-.

In einer ersten und zugleich bevorzugten Ausführungsform zwei- oder mehrkerniger Platinkomplexe vom Typ [L1L2Pt[O(CO)R1]X]ₙ bedeuten L1 und L2 zusammen eine verschiedene Platinzentren verbrückende als Diolefinligand fungierende Verbindung L1L2; X bedeutet Bromid, Chlorid, lodid oder -O(CO)R2; n bedeutet 2, 3, 4 oder 5, bevorzugt 2; und - O(CO)R1 und -O(CO)R2 bedeuten gleiche oder verschiedene nichtaromatische C6-C18- oder bevorzugt C8-C18-Monocarbonsäurereste, Phenylessigsäurereste bevorzugt jeweils ausgenommen.

In einer zweiten Ausführungsform zwei- oder mehrkerniger Platinkomplexe vom Typ [L1L2Pt[O(CO)R1]X]ₙ bedeuten L1 und L2 zusammen eine verschiedene Platinzentren verbrückende als Diolefinligand fungierende Verbindung L1L2; X bedeutet -O(CO)R2; n bedeutet 2, 3, 4 oder 5, bevorzugt 2; und -O(CO)R1 und -O(CO)R2 bedeuten zusammen einen nichtaromatischen verschiedene Platinzentren verbrückenden C8-C18-Dicarbonsäurerest - O(CO)R1R2(CO)O-.

In einer dritten Ausführungsform zwei- oder mehrkerniger Platinkomplexe vom Typ [L1L2Pt[O(CO)R1]X]ₙ bedeuten L1 und L2 gleiche oder verschiedene Monoolefinliganden; X bedeutet -O(CO)R2; n bedeutet 2, 3, 4 oder 5, bevorzugt 2; und -O(CO)R1 und -O(CO)R2 bedeuten zusammen einen nichtaromatischen verschiedene Platinzentren verbrückenden C8-C18-Dicarbonsäurerest -O(CO)R1 R2(CO)O-.

Besagte Platinkomplexe können in der erfindungsgemäßen Zubereitung in individualisierter aber auch in vergesellschafteter Form vorliegen, also alleine oder auch als Gemisch mehrerer verschiedener Spezies jeweils vom Typ [L1L2Pt[O(CO)R1]X]ₙ.

L1 und L2 bedeuten alleine gleiche oder verschiedene, bevorzugt gleiche Monoolefine, oder zusammen eine mehrfach olefinisch ungesättigte Verbindung L1L2, beispielsweise ein Diolefin oder ein Polyolefin, das als Diolefinligand fungieren kann. Bevorzugt sind dabei die mehrfach olefinisch ungesättigten Verbindungen L1L2, welche als Diolefinliganden fungieren können.

Beispiele für Monoolefine umfassen C2-C18-Kohlenwasserstoffe mit einer einzigen olefinisch ungesättigten Doppelbindung. Dabei kann es sich um lineare Verbindungen, verzweigte Verbindungen oder Verbindungen mit cyclischen Strukturen handeln. Bevorzugt handelt es sich um reine Kohlenwasserstoffe; die Anwesenheit von Heteroatomen beispielsweise auch in Form funktioneller Gruppen ist jedoch auch möglich. Bevorzugte Beispiele für Monoolefine umfassen Ethen, Propen und Cyclohexen.

Beispiele für Diolefine respektive für Verbindungen vom Typ L1L2, welche befähigt sind als Diolefinligand zu fungieren, umfassen Kohlenwasserstoffe wie COD (1,5-Cyclooctadien), NBD (Norbornadien), COT (Cyclooctatetraen) und 1,5-Hexadien, insbesondere COD und NBD. Bevorzugt handelt es sich um reine Kohlenwasserstoffe; die Anwesenheit von Heteroatomen beispielsweise auch in Form funktioneller Gruppen ist jedoch auch möglich.

X kann Bromid, Chlorid, lodid oder -O(CO)R2 bedeuten, bevorzugt bedeutet es Chlorid oder - O(CO)R2, insbesondere -O(CO)R2.

Die jeweils nichtaromatischen Monocarbonsäurereste -O(CO)R1 und -O(CO)R2 bedeuten alleine gleiche oder verschiedene nichtaromatische C6-C18- oder bevorzugt C8-C18-Monocarbonsäurereste, bevorzugt jeweils mit Ausnahme eines Phenylessigsäurerests, oder sie bedeuten zusammen einen nichtaromatischen C8-C18-Dicarbonsäurerest vom Typ - O(CO)R1R2(CO)O-. Der in diesem Zusammenhang verwendete Begriff "nichtaromatisch" schließt rein aromatische Monocarbonsäure- und Dicarbonsäurereste aus, nicht jedoch araliphatische Monocarbon- und Dicarbonsäurereste, deren Carboxylfunktion(en) an aliphatischen Kohlenstoff gebunden ist/sind. -O(CO)R1 als auch -O(CO)R2 bedeuten jeweils bevorzugt keinen Phenylessigsäurerest. Bevorzugt bedeuten -O(CO)R1 und -O(CO)R2 gleiche oder verschiedene nichtaromatische C6-C18- oder insbesondere C8-18-Monocarbonsäurereste bevorzugt jeweils mit Ausnahme eines Phenylessigsäurerests; insbesondere bevorzugt bedeuten -O(CO)R1 und -O(CO)R2 gleiche nichtaromatische C6-C18- oder insbesondere C8-C18-Monocarbonsäurereste und dabei bevorzugt keine Phenylessigsäurereste.

Beispiele für nichtaromatische C6-C18- oder die bevorzugten C8-C18-Monocarbonsäuren mit Resten -O(CO)R1 respektive -O(CO)R2 umfassen die isomeren Hexansäuren einschließlich n-Hexansäure, die isomeren Heptansäuren einschließlich n-Heptansäure, die isomeren Octansäuren einschließlich n-Octansäure und 2-Ethylhexansäure, die isomeren Nonansäuren einschließlich n-Nonansäure, und die isomeren Decansäuren einschließlich n-Decansäure, um nur einige Beispiele zu nennen. Es sind nicht nur lineare Vertreter sondern auch solche mit Verzweigungen und/oder cyclischen Strukturen umfasst, wie beispielsweise 2-Ethylhexansäure, Cyclohexancarbonsäure und Neodecansäure. Die jeweils an eine Carboxylgruppe gebundenen Reste R1 und R2 umfassen 5 bis 17 respektive 7 bis 17 Kohlenstoffatome; Benzylreste sind dabei jeweils bevorzugt ausgenommen.

Beispiele für nichtaromatische C8-C18-Dicarbonsäuren vom Typ HOOCR1R2COOH umfassen entsprechend substituierte Malonsäuren, entsprechend substituierte 1,1-Cyclobutandicarbonsäuren, Cyclohexandicarbonsäuren, um nur einige Beispiele zu nennen. Das zwei Carboxylgruppen tragende Strukturelement -R1R2- umfasst 6 bis 16 Kohlenstoffatome.

Bevorzugte Beispiele für Platinkomplexe (B) umfassen [(COD)Pt[O(CO)R1]₂]ₙ und [(NBD)Pt[O(CO)R1]₂]ₙ, wobei n 1 oder 2 und insbesondere 1 ist, und wobei R1 für einen nichtaromatischen C5-C17- respektive C7-C17-Kohlenwasserstoffrest steht, bevorzugt mit Ausnahme eines Benzylrests.

Die Platinkomplexe [L1L2Pt[O(CO)R1]X]ₙ können auf einfachem Wege durch Ligandenaustausch hergestellt werden, insbesondere ohne dabei Carbonsäuresalze des Silbers zu verwenden. Das Herstellungsverfahren umfasst ein Durchmischen respektive Suspendieren oder Emulgieren eines Zweiphasensystems. Die eine Phase umfasst dabei ein Edukt vom Typ [L1L2PtX₂]ₙ mit X ausgewählt unter Bromid, Chlorid und lodid, bevorzugt Chlorid, entweder als solches oder in Form einer zumindest im Wesentlichen nicht wassermischbaren organischen Lösung eines solchen Eduktes. Bevorzugte Edukte umfassen [L1L2PtCl₂]ₙ mit n als ganzer Zahl von 1 bis 5, insbesondere mit n = 1. Beispiele für geeignete zumindest im Wesentlichen nicht wassermischbare organische Lösemittel umfassen neben Aromaten und chlorierten Kohlenwasserstoffen wie Toluol, Xylol, Di-, Tri- und Tetrachlormethan auch sauerstoffhaltige Lösemittel, beispielsweise entsprechende nicht wassermischbare Ketone, Ester und Ether. Die andere Phase hingegen umfasst eine wässrige Lösung von Alkalisalz (insbesondere Natrium- oder Kaliumsalz) und/oder von Magnesiumsalz einer C6-C18- respektive C8-C18-Monocarbonsäure vom Typ R1COOH sowie gegebenenfalls zusätzlich vom Typ R2COOH oder entsprechendes Alkali- und/oder Magnesiumsalz einer C8-C18-Dicarbonsäure vom Typ HOOCR1R2COOH. Die Wahl der Art des oder der Carbonsäuresalze richtet sich nach der Art des herzustellenden Platinkomplexes oder der herzustellenden Gesellschaft von Platinkomplexen. Die beiden Phasen werden intensiv unter Bildung einer Suspension oder Emulsion durchmischt, beispielsweise durch Schütteln und/oder Rühren. Das Durchmischen wird zwecks Aufrechterhaltung des Suspensions- oder Emulsionszustandes beispielsweise für eine Zeitdauer von 0,5 bis 24 Stunden durchgeführt, beispielsweise bei einer Temperatur im Bereich von 20 bis 50°C. Dabei findet der Ligandenaustausch statt, wobei sich der oder die gebildeten Platinkomplexe in der organischen Phase lösen, während sich das ebenfalls gebildete AlkaliX-Salz oder MgX₂-Salz in der wässrigen Phase löst. Nach beendetem Suspendieren oder Emulgieren werden organische und wässrige Phase voneinander getrennt. Aus der organischen Phase können der oder die gebildeten Platinkomplexe gewonnen und gegebenenfalls anschließend mittels üblicher Methoden gereinigt werden.

So kann beispielsweise, um nur ein konkretes Beispiel zu nennen, (COD)Pt[O(CO)CH(C₂H₅)C₄H₉]₂ durch gemeinsames Emulgieren einer Lösung von (COD)PtCl₂ in Dichlormethan mit einer wässrigen Lösung von Natrium-2-ethylhexanoat hergestellt werden. Nach beendetem Emulgieren kann die dabei durch Ligandenaustausch gebildete Kochsalzlösung von der Dichlormethanphase abgetrennt und aus letzterer das (COD)Pt[O(CO)CH(C₂H₅)C₄H₉]₂ isoliert und gegebenenfalls durch übliche Reinigungsverfahren aufgereinigt werden. Analog kann beispielsweise bei entsprechend gewählter Stöchiometrie auch der Platinkomplex (COD)Pt[O(CO)CH(C₂H₅)C₄H₉]Cl hergestellt werden.

Eine wichtige Eigenschaft neben vorerwähnter Löslichkeit in üblichen organischen Lösemitteln ist die vergleichsweise niedrige Zersetzungstemperatur des oder der Platinkomplexe (B), beispielsweise schon ab 150°C bis 250°C, häufig nicht höher als 200°C. Diese Eigenschaftskombination erlaubt es, solche Platinkomplexe als Bestandteil (B) der erfindungsgemäßen Zubereitung zur Herstellung von Platinschichten auf Substraten zu verwenden; bei dieser Art der Verwendung stellt die erfindungsgemäße Zubereitung ein Überzugsmittel (Beschichtungsmittel) dar, d.h. sie ist dann als Überzugsmittel zubereitet und verwendbar.

Die erfindungsgemäße Zubereitung enthält 0 bis 10 Gew.-%, bevorzugt 0 bis 3 Gew.-% mindestens eines Additivs (C). Die erfindungsgemäße Zubereitung kann demzufolge additivfrei sein oder bis zu 10 Gew.-% mindestens eines Additivs enthalten. Beispiele für Additive umfassen Benetzungsadditive, Rheologieadditive, Entschäumer, Entlüfter, Additive zur Beeinflussung der Oberflächenspannung und Geruchsstoffe.

Erfindungsgemäße Zubereitungen können durch einfaches Vermischen der Bestandteile (A), (B) und, falls gewünscht, (C) hergestellt werden. Der Fachmann wählt dabei das Mengenverhältnis der Bestandteile angepasst an den jeweiligen Verwendungszweck und/oder die dabei zur Anwendung kommende Applikationsmethode aus.

Die erfindungsgemäßen Zubereitungen können zur Herstellung von Platinschichten auf Substraten, insbesondere auch auf temperaturempfindlichen Substraten verwendet werden. Die erfindungsgemäßen Zubereitungen können dabei zunächst zur Herstellung von Überzugsschichten (Beschichtungen) verwendet werden, welche anschließend einer thermischen Zersetzung unterworfen werden können. Bei der thermischen Behandlung zersetzen sich die Überzugsschichten unter Platinbildung, d.h. die Überzugsschichten werden letztendlich in Platinschichten überführt. Die Erfindung betrifft daher auch ein Verfahren zur Herstellung einer Platinschicht auf einem Substrat, umfassend die Schritte:
(1) Applikation einer Überzugsschicht aus einer erfindungsgemäßen Zubereitung auf ein Substrat, und
(2) thermische Zersetzung der Überzugsschicht unter Ausbildung einer Platinschicht.

Bei den in Schritt (1) mit der Überzugsschicht zu versehenden Substraten kann es sich um Substrate handeln, die verschiedenste Materialien umfassen. Die Substrate können dabei nur ein oder auch mehrere Materialien umfassen. Beispiele für Materialien umfassen unter anderem Glas; Carbidsubstrate wie beipielsweise Titancarbid, Molybdäncarbid, Wolframcarbid, Siliziumcarbid; Nitridsubstrate wie beispielsweise Aluminiumnitrid, Titannitrid, Siliziumnitrid; Boridsubstrate wie beispielsweise Titanborid, Zirkonborid; Keramiksubstrate einschließlich solcher auf Basis von oxidischer Keramik und solcher, wie sie als Katalysatorträger üblich sind; Halbleitersubstrate wie beispielsweise Siliziumsubstrate; Metall; Kunststoff; modifizierte oder unmodifizierte Polymere natürlichen Ursprungs; Kohlenstoffsubstrate; Holz; Karton und Papier. Die Substrate können auf inneren und/oder äußeren Oberflächen oder auf inneren und/oder äußeren Oberflächenanteilen mit der Überzugsschicht versehen werden.

Bei der Herstellung der Überzugsschicht gemäß Schritt (1) können an sich bekannte Applikationsmethoden zur Anwendung gelangen.

Eine erste Applikationsmethode ist das Tauchen. Dabei wird das mit der Überzugsschicht respektive das final mit der Platinschicht zu versehende Substrat in die erfindungsgemäße Zubereitung ein- und wieder ausgetaucht. Bevorzugt liegt der Anteil des Bestandteils (A) beim Tauchen im Bereich von 30 bis 90 Gew.-% der erfindungsgemäßen Zubereitung und der des Bestandteils (B) im Bereich von 10 bis 70 Gew.-%.

Eine zweite Applikationsmethode ist der Sprühauftrag. Dabei wird das mit der Überzugsschicht respektive das final mit der Platinschicht zu versehende Substrat mit der erfindungsgemäßen Zubereitung unter Verwendung eines üblichen Sprühbeschichtungswerkzeugs sprühbeschichtet. Beispiele für Sprühbeschichtungswerkzeuge sind pneumatische Spritzpistolen, Airless-Spritzpistolen, Rotationszerstäuber oder ähnliches. Bevorzugt liegt der Anteil des Bestandteils (A) beim Sprühauftrag im Bereich von 50 bis 90 Gew.-% der erfindungsgemäßen Zubereitung und der des Bestandteils (B) im Bereich von 10 bis 50 Gew.-%.

Eine dritte Applikationsmethode ist das Drucken. Dabei wird das mit der Überzugsschicht respektive das final mit der Platinschicht zu versehende Substrat mit der erfindungsgemäßen Zubereitung bedruckt. Ein bevorzugtes Druckverfahren ist dabei das Inkjetdrucken. Ein weiteres bevorzugtes Druckverfahren ist dabei der Siebdruck. Bevorzugt liegt der Anteil des Bestandteils (A) beim Drucken im Bereich von 50 bis 90 Gew.-% der erfindungsgemäßen Zubereitung und der des Bestandteils (B) im Bereich von 10 bis 50 Gew.-%.

Eine vierte Applikationsmethode ist der Auftrag mittels eines mit der erfindungsgemäßen Zubereitung getränkten Auftragungswerkzeugs, beispielsweise ein Pinsel, eine Bürste, ein Filz oder ein Tuch. Dabei wird die erfindungsgemäße Zubereitung vom Auftragungswerkzeug auf das mit der Überzugsschicht respektive das final mit der Platinschicht zu versehende Substrat übertragen. Bevorzugt liegt der Anteil des Bestandteils (A) bei einer derartigen Auftragungstechnik im Bereich von 30 bis 90 Gew.-% der erfindungsgemäßen Zubereitung und der des Bestandteils (B) im Bereich von 10 bis 70 Gew.-%.

Die aus der erfindungsgemäßen Zubereitung aufgebrachte, den mindestens einen Platinkomplex vom Typ [L1L2Pt[O(CO)R1]X]ₙ umfassende Überzugsschicht kann zunächst getrocknet und dabei teilweise oder vollständig vom organischen Lösemittel befreit werden, bevor sie bzw. der getrocknete Rückstand einer thermischen Zersetzung unter Bildung metallischen Platins in Form einer Schicht unterworfen wird. Die zwecks thermischer Zersetzung erfolgende thermische Behandlung umfasst eine Erwärmung auf eine Objekttemperatur oberhalb der Zersetzungstemperatur des mindestens eines Platinkomplexes.

Bei Vorhandensein mehrerer verschiedener Platinkomplexe vom Typ [L1L2Pt[O(CO)R1]X]ₙ wird der Fachmann die Objekttemperatur oberhalb der Zersetzungstemperatur des Platinkomplexes vom Typ (B) mit der höchsten Zersetzungstemperatur wählen. Im Allgemeinen wird dazu beispielsweise kurzzeitig auf eine Objekttemperatur oberhalb der Zersetzungstemperatur erwärmt, beispielsweise für einen Zeitraum von 1 Minute bis 30 Minuten auf eine Objekttemperatur im Bereich von 150°C bis 200°C oder von 150 bis 250°C oder höher, beispielsweise bis 1000°C. Das Erwärmen kann insbesondere in einem Ofen und/oder durch Infrarotbestrahlung erfolgen. Im Allgemeinen wird eine Objekttemperatur geringfügig oberhalb der betreffenden Zersetzungstemperatur gewählt. Im Allgemeinen dauert das Erwärmen, genauer gesagt das Einhalten der Objekttemperatur nicht länger als 15 Minuten.

Vorteilhaft bei der Herstellung von Platinschichten mittels der erfindungsgemäßen Zubereitungen ist auch, dass keine kolloidales Platin oder Nanoplatin enthaltende Zubereitungen verwendet werden müssen, so dass damit verbundene eventuelle Risiken vermieden werden können. Außerdem kann bei der zweiten und dritten der vorerwähnten Applikationsmethoden durch die Verwendung der erfindungsgemäßen Zubereitung ein Verstopfen der Applikationswerkzeuge, genauer gesagt das Verstopfen feiner Öffnungen oder Düsen von Sprühauftragswerkzeugen bzw. Inkjetdüsen vermieden werden; schließlich stellt sich die Frage antrocknenden oder aggregierenden kolloidalen Platins oder Nanoplatins hier nicht.

Die so erhältlichen Platinschichten zeichnen sich durch hohen metallischen Glanz vergleichbar einem Spiegel aus, vorausgesetzt man arbeitet mit Substraten mit glatten, nicht zu rauen Oberflächen; die Platinschichten sind homogen im Sinne einer glatten nichtkörnigen äußeren Oberfläche. Die Dicke der mit dem erfindungsgemäßen Verfahren erhältlichen Platinschichten kann beispielsweise im Bereich von 50 nm bis 5 µm liegen und die Platinschichten können eine flächige Natur mit oder ohne gewünschte Unterbrechungen innerhalb der Fläche haben oder ein gewünschtes Muster oder Design aufweisen. Wie aus den vorerwähnten Beispielen für Substrate ersichtlich, können die Platinschichten sogar auf temperaturempfindlichen Substraten erzeugt werden, d.h. beispielsweise auf Substraten, die oberhalb 200°C nicht temperaturstabil sind; beispielsweise kann es sich um temperaturempfindliche Polymersubstrate handeln, beispielsweise solche auf Polyolefin- oder Polyesterbasis.

### Beispiele

### Beispiel 1 (Ausrüstung eines Objektträgers aus Glas mit einer Platinschicht):

Eine Lösung von 65 mmol (COD)PtCl₂ in 100 ml Dichlormethan wurde gerührt und eine Lösung von 260 mmol Natrium-2-isodecanoat in 500 ml Wasser zugegeben. Die Zweiphasenmischung wurde 24 h bei 20°C durch intensives Rühren emulgiert. Dabei färbte sich die Dichlormethanphase gelb.

Die Dichlormethanphase wurde abgetrennt und das Lösungsmittel abdestilliert. Der zähflüssige, gelbe Rückstand wurde in 150 ml Petroleumbenzin (40-60) aufgenommen und die Lösung mit Magnesiumsulfat getrocknet und filtriert. Dann wurde das Petroleumbenzin vollständig abdestilliert. Zurück blieb ein zähflüssiger gelber Rückstand von (COD)Pt[O(CO)(CH₂)₅C(CH₃)₃]₂.

10 g des gelben Rückstandes wurden in 20 g einer Lösemittel-/Additivmischung (50 Gew.-% Ethanol, 49,9 Gew.-% Propylenglykolmonopropylether, 0,1 Gew.-% BYK-333 (Oberflächenadditiv von BYK) gelöst. Die 10 Gew.-% Platin enthaltende Lösung wurde mittels einer Airbrushsprühpistole auf einen Objektträger aus Glas gesprüht. Der beschichtete Glasobjektträger wurde in einem Laborofen auf eine Objekttemperatur von 200°C aufgeheizt und für 15 Minuten bei dieser Temperatur gehalten. Auf dem Objektträger hatte sich eine glänzende elektrisch leitfähige Schicht aus Platin gebildet.

### Beispiele 2a bis 2d (Ausrüstung von Aluminiumoxidplättchen mit einer Platinschicht):

2a: In die Lösung aus Beispiel 1 wurde ein unglasiertes Plättchen aus Aluminiumoxid (50 mm x 50 mm) getaucht und nach dem Austauchen in einem Laborofen auf eine Objekttemperatur von 200°C aufgeheizt und für 15 Minuten bei dieser Temperatur gehalten. Auf dem Plättchen hatte sich eine glänzende elektrisch leitfähige Schicht aus Platin gebildet.

2b: Versuch 2a wurde mit einer Objekttemperatur von 900°C wiederholt unter Erhalt eines vergleichbaren Resultats.

2c und 2d: Die Versuche 2a und 2b wurden mit einem glasierten Plättchen aus Aluminiumoxid wiederholt unter Erhalt jeweils vergleichbarer Resultate, hier jedoch, abweichend vom Resultat der Versuche 2a und 2b, in Form spiegelnder elektrisch leitfähiger Schichten aus Platin.

### Beispiel 3 (Ausrüstung eines Polyurethanschlauches mit einer Platinschicht):

Ein 5 mm dicker Schlauch aus Polyurethan wurde dreimal hintereinander durch ein Filzstückchen gezogen, welches mit der Lösung aus Beispiel 1 getränkt war. Der so beschichtete Schlauch wurde in einem Laborofen auf eine Objekttemperatur von 175°C aufgeheizt und für 5 Minuten bei dieser Temperatur gehalten. Auf dem Schlauch hatte sich eine glänzende elektrisch leitfähige Schicht aus Platin gebildet.

### Beispiel 4 (Ausrüstung einer Polyimidfolie mit einer gemusterten Platinschicht):

Eine Kapton^{®}-Folie (Polyimid) wurde mithilfe eines Inkjetdruckers bei einer Auflösung von 1270 dpi in einem Mäander-Design mit der Lösung aus Beispiel 1 bedruckt. Die so bedruckte Folie wurde in einem Laborofen auf eine Objekttemperatur von 200°C aufgeheizt und für 5 Minuten bei dieser Temperatur gehalten. Auf der Folie hatte sich eine glänzende elektrisch leitfähige Schicht aus Platin in Gestalt des Mäander-Designs mit einer Breite der Leiterbahnen von 2,5 mm gebildet.

### Beispiel 5 (Ausrüstung eines Objektträgers aus Glas mit einer Platinschicht):

Analog zu Beispiel 1 wurden 32,5 mmol (COD)PtCl₂ in 100 ml Dichlormethan mit 130 mmol Natriumcyclohexanoat in 200 ml Wasser umgesetzt. Man erhielt einen gelben Rückstand von (COD)Pt[O(CO)C₆H₁₁]₂.

2 g des gelben Rückstandes wurden in 4.86 g Dipropylenglykolmonopropylether gelöst. In diese 10 Gew.-% Platin enthaltende Lösung wurde ein Objektträger aus Glas getaucht und nach dem Austauchen in einem Laborofen auf eine Objekttemperatur von 200°C aufgeheizt und für 15 Minuten bei dieser Temperatur gehalten. Auf dem Objektträger hatte sich eine glänzende elektrisch leitfähige Schicht aus Platin gebildet.

### Beispiel 6 (Ausrüstung einer Polyimidfolie mit einer Platinschicht):

Analog zu Beispiel 1 wurden 65 mmol (COD)PtCl₂ in 100 ml Dichlormethan mit 260 mmol Natrium-2-ethylhexanoat in 500 ml Wasser umgesetzt. Man erhielt einen gelben Rückstand von (COD)Pt[O(CO)CH(C₂H₅)C₄H₉]₂.

10 g des gelben Rückstandes wurden in einer Mischung aus 15 g Propylenglykolmonopropylether und 15 g Ethanol gelöst. In diese 8,2 Gew.-% Platin enthaltende Lösung wurde ein 10 x 40 mm Streifen einer Kapton^{®}-Folie getaucht und nach dem Austauchen in einem Laborofen auf eine Objekttemperatur von 200°C aufgeheizt und für 3 Minuten bei dieser Temperatur gehalten. Auf der Folie hatte sich eine glänzende elektrisch leitfähige Schicht aus Platin gebildet.

### Beispiel 7 (Ausrüstung eines glasierten Aluminiumoxidplättchens mit einer Platinschicht):

In die Lösung aus Beispiel 6 wurde ein glasiertes Plättchen aus Aluminiumoxid getaucht und nach dem Austauchen in einem Laborofen auf eine Objekttemperatur von 200°C aufgeheizt und für 5 Minuten bei dieser Temperatur gehalten. Auf dem Plättchen hatte sich eine glänzende elektrisch leitfähige Schicht aus Platin gebildet.

### Beispiel 8 (Ausrüstung einer Polyimidfolie mit einer Platinschicht):

Analog zu Beispiel 1 wurden 27,3 mmol (NBD)PtCl₂ in 100 ml Dichlormethan mit 110 mmol Natrium-2-ethylhexanoat in 100 ml Wasser umgesetzt. Man erhielt einen gelben Rückstand von (NBD)Pt[O(CO)CH(C₂H₅)C₄H₉]₂, welcher analog zu Beispiel 6 weiterverarbeitet wurde unter Erhalt einer mit einer glänzenden elektrisch leitfähigen Schicht aus Platin versehenen Kapton^{®}-Folie.

## Patentansprüche

1. Zubereitung enthaltend oder bestehend aus:
(A) 30 bis 90 Gew.-% mindestens eines organischen Lösemittels,
(B) 10 bis 70 Gew.-% mindestens eines Platinkomplexes vom Typ [L1L2Pt[O(CO)R1]X]ₙ,
wobei L1 und L2 gleiche oder verschiedene Monoolefinliganden oder zusammen eine als Diolefinligand fungierende Verbindung L1L2 bedeuten,
wobei X ausgewählt ist unter Bromid, Chlorid, lodid und -O(CO)R2,
wobei -O(CO)R1 und -O(CO)R2 gleiche oder verschiedene nichtaromatische C6-C18- oder C8-C18-Monocarbonsäurereste oder zusammen einen nichtaromatischen C8-C18-Dicarbonsäurerest -O(CO)R1R2(CO)O- bedeuten,
wobei es sich um einkernige Platinkomplexe mit n = 1 handelt oder wobei es sich im Falle der Anwesenheit von L1L2 und/oder von -O(CO)R1R2(CO)O- um mehrkernige Platinkomplexe mit ganzzahligem n > 1 handeln kann, und
(C) 0 bis 10 Gew.-% mindestens eines Additivs.

2. Zubereitung nach Anspruch 1, wobei L1L2 eine als Diolefinligand fungierende Verbindung bedeutet,
wobei X ausgewählt ist unter Bromid, Chlorid, lodid und -O(CO)R2,
wobei -O(CO)R1 und -O(CO)R2 gleiche oder verschiedene nichtaromatische C6-C18- oder C8-C18-Monocarbonsäurereste bedeuten, und
wobei es sich um einkernige Platinkomplexe mit n = 1 oder um mehrkernige Platinkomplexe mit ganzzahligem n > 1 handelt.

3. Zubereitung nach Anspruch 1 oder 2, wobei ganzzahliges n > 1 im Bereich von 2 bis 5 liegt.

4. Zubereitung nach einem der vorhergehenden Ansprüche in Form einer nichtkolloidalen organischen Lösung.

5. Zubereitung nach einem der vorhergehenden Ansprüche mit einem dem mindestens einen Platinkomplex entstammenden Platingehalt im Bereich von 2,5 bis 25 Gew.-%.

6. Zubereitung nach einem der vorhergehenden Ansprüche, wobei der mindestens eine Platinkomplex die Formel [(COD)Pt[O(CO)R1]₂]ₙ oder [(NBD)Pt[O(CO)R1]₂]ₙ hat, wobei n 1 oder 2 ist, wobei COD für 1,5-Cyclooctadien und wobei R1 für einen nichtaromatischen C5-C17- oder C7-C17-Kohlenwasserstoffrest steht.

7. Zubereitung nach einem der vorhergehenden Ansprüche, wobei die Zersetzungstemperatur des mindestens einen Platinkomplexes im Bereich von 150 bis 200 oder von 150 bis 250°C liegt.

8. Zubereitung nach einem der vorhergehenden Ansprüche, wobei das mindestens eine Additiv (C) ausgewählt ist aus der Gruppe bestehend aus Benetzungsadditiven, Rheologieadditiven, Entschäumern, Entlüftern, Additiven zur Beeinflussung der Oberflächenspannung und Geruchsstoffen.

9. Verfahren zur Herstellung einer Platinschicht auf einem Substrat, umfassend die Schritte:
(1) Applikation einer Überzugsschicht aus einer Zubereitung nach einem der vorhergehenden Ansprüche auf ein Substrat, und
(2) thermische Zersetzung der Überzugsschicht unter Ausbildung einer Platinschicht.

10. Verfahren nach Anspruch 9, wobei das Substrat ein oder mehrere Materialien ausgewählt aus der Gruppe bestehend aus Glas, Keramik, Halbleitersubstraten, Metall, Kunststoff, modifizierten oder unmodifizierten Polymeren natürlichen Ursprungs, Kohlenstoffsubstraten, Karton und Papier umfasst.

11. Verfahren nach Anspruch 9 oder 10, wobei das Substrat auf inneren und/oder äußeren Oberflächen oder auf inneren und/oder äußeren Oberflächenanteilen mit der Überzugsschicht versehen wird.

12. Verfahren nach einem der Ansprüche 9 bis 11, wobei die zur Herstellung der Überzugsschicht verwendete Applikationsmethode ausgewählt ist aus der Gruppe bestehend aus Tauchen, Sprühauftrag, Drucken, Auftrag mittels Pinsel, Auftrag mittels Bürste, Auftrag mittels Filz und Auftrag mittels Tuch.

13. Verfahren nach einem der Ansprüche 9 bis 12, wobei die in Schritt (1) aufgebrachte Überzugsschicht zunächst getrocknet und dabei teilweise oder vollständig vom organischen Lösemittel befreit wird, bevor sie in Schritt (2) der thermischen Zersetzung unterworfen wird.

14. Verfahren nach einem der Ansprüche 9 bis 13, wobei die thermische Zersetzung gemäß Schritt (2) durch thermische Behandlung erfolgt, welche eine Erwärmung auf eine Objekttemperatur oberhalb der Zersetzungstemperatur des mindestens einen Platinkomplexes umfasst.

15. Verfahren nach Anspruch 14, wobei die Erwärmung auf die Objekttemperatur in einem Ofen und/oder durch Infrarotbestrahlung erfolgt.

16. Verfahren nach einem der Ansprüche 9 bis 15, wobei die Platinschicht 50 nm bis 5 µm dick ist.

## Claims

1. A preparation containing or consisting of:
(A) 30 to 90 % by weight of at least one organic solvent;
(B) 10 to 70 % by weight of at least one platinum complex of the type
[L1L2Pt[O(CO)R1]X]ₙ,
wherein L1 and L2 represent the same or different monoolefin ligands or together represent a compound L1L2 acting as a diolefin ligand,
wherein X is selected from bromide, chloride, iodide, and -O(CO)R2,
wherein -O(CO)R1 and -O(CO)R2 represent the same or different C6-C18 or C8-C18 non-aromatic monocarboxylic acid groups, or together represent a C8-C18 non-aromatic dicarboxylic acid group -O(CO)R1R2(CO)O-,
wherein the platinum complexes are mononuclear platinum complexes with n = 1, or wherein, if L1L2 and/or -O(CO)R1R2(CO)O- are present, the platinum complexes may be polynuclear platinum complexes with a whole number n > 1, and
(C) 0 to 10 % by weight of at least one additive.

2. The preparation according to claim 1, wherein L1L2 represents a compound acting as a diolefin ligand,
wherein X is selected from bromide, chloride, iodide, and -O(CO)R2,
wherein -O(CO)R1 and -O(CO)R2 represent the same or different C6-C18 or C8-C18 non-aromatic monocarboxylic acid groups, and
wherein the platinum complexes are mononuclear platinum complexes with n = 1, or polynuclear platinum complexes with a whole number n > 1.

3. The preparation according to either claim 1 or claim 2, wherein whole number n > 1 is in the range of 2 to 5.

4. The preparation according to any of the preceding claims in the form of a non-colloidal organic solution.

5. The preparation according to any of the preceding claims, having a platinum content originating from the at least one platinum complex in the range of 2.5 to 25 % by weight.

6. The preparation according to any of the preceding claims, wherein the at least one platinum complex has the formula [(COD)Pt[O(CO)R1]₂]ₙ or [(NBD)Pt[O(CO)R1]₂]ₙ, wherein n is 1 or 2, wherein COD stands for 1,5-Cyclooctadiene and wherein R1 stands for a C5-C17 or C7-C17 non-aromatic hydrocarbon group.

7. The preparation according to any of the preceding claims, wherein the decomposition temperature of the at least one platinum complex is in the range of 150 to 200 or 150 to 250 °C.

8. The preparation according to any of the preceding claims, wherein the at least one additive (C) is selected from the group consisting of wetting additives, rheological additives, defoamers, deaerators, additives for influencing the surface tension and odorants.

9. A method for producing a platinum layer on a substrate, comprising the steps of:
(1) applying a coating layer of a preparation according to any of the preceding claims to a substrate, and
(2) thermally decomposing the coating layer to form a platinum layer.

10. The method according to claim 9, wherein the substrate comprises one or more materials selected from the group consisting of glass, ceramic, semiconductor substrates, metal, plastics material, modified or unmodified polymers of natural origin, carbon substrates, card and paper.

11. The method according to either claim 9 or claim 10, wherein the substrate is provided with the coating layer on inner and/or outer surfaces or on inner and/or outer surface portions.

12. The method according to any of claims 9 to 11, wherein the application method used to produce the coating layer is selected from the group consisting of dipping, spray application, printing, application by means of paintbrush, application by means of brush, application by means of felt, and application by means of cloth.

13. The method according to any of claims 9 to 12, wherein the coating layer applied in step (1) is initially dried and in the process partially or completely freed of the organic solvent before it is subjected to thermal decomposition in step (2).

14. The method according to any of claims 9 to 13, wherein the thermal decomposition according to step (2) takes place by thermal treatment, which comprises heating to an object temperature above the decomposition temperature of the at least one platinum complex.

15. The method according to claim 14, wherein the heating to the object temperature takes place in a kiln and/or by infrared irradiation.

16. The method according to any of claims 9 to 15, wherein the platinum layer is 50 nm to 5 µm thick.

## Revendications

1. Préparation contenant ou constituée de :
(A) 30 à 90 % en poids d'au moins un solvant organique,
(B) 10 à 70 % en poids d'au moins un complexe de platine du type [L1L2Pt[O(CO)R1]X]ₙ,
où L1 et L2 représentent des ligands mono-oléfine identiques ou différents ou conjointement un composé faisant office de ligand dioléfine L1L2,
où X est choisi parmi le bromure, le chlorure, l'iodure et -O(CO)R2,
où -O(CO)R1 et -O(CO)R2 représentent des résidus acide monocarboxylique en C6-C18 ou en C8-C18 non aromatiques identiques ou différents ou conjointement un résidu d'acide dicarboxylique en C8-C18 non aromatique -O(CO)R1R2(CO)O-,
où il peut s'agir de complexes de platine mononucléaires avec n = 1 ou où il peut s'agir, en cas de présence de L1L2 et/ou de -O(CO)R1R2(CO)O-, de complexes de platine polynucléaires avec un entier n > 1, et
(C) 0 à 10 % en poids d'au moins un additif.

2. Préparation selon la revendication 1, dans laquelle L1L2 représente un composé faisant office de ligand dioléfine,
où X est choisi parmi le bromure, le chlorure, l'iodure et -O(CO)R2,
où -O(CO)R1 et -O(CO)R2 représentent des résidus d'acide monocarboxylique en C6-C18 ou C8-C18 non aromatiques identiques ou différents, et
où il s'agit de complexes de platine mononucléaires avec n = 1 ou de complexes de platine polynucléaires avec un entier n > 1.

3. Préparation selon la revendication 1 ou 2, dans laquelle l'entier n > 1 se trouve dans la plage de 2 à 5.

4. Préparation selon l'une quelconque des revendications précédentes, sous forme d'une solution organique non colloïdale.

5. Préparation selon l'une quelconque des revendications précédentes, comportant une teneur en platine, provenant de l'au moins un complexe de platine, dans la plage de 2,5 à 25 % en poids.

6. Préparation selon l'une quelconque des revendications précédentes, dans laquelle l'au moins un complexe de platine a la formule [(COO)Pt[O(CO)R1]₂]ₙ ou [(NBD)Pt[O(CO)R1]₂]ₙ, où n est 1 ou 2, où COD représente le 1,5-cyclooctadiène et où R1 représente un radical hydrocarboné en C5-C17 ou C7-C17 non aromatique.

7. Préparation selon l'une quelconque des revendications précédentes, dans laquelle la température de décomposition de l'au moins un complexe de platine se trouve dans la plage de 150 à 200 ou de 150 à 250 °C.

8. Préparation selon l'une quelconque des revendications précédentes, dans laquelle l'au moins un additif (C) est choisi dans le groupe constitué par des additifs de mouillage, des additifs rhéologiques, des agents antimousse, des aérateurs, des additifs influençant la tension superficielle et des substances odorantes.

9. Procédé de réalisation d'une couche de platine sur un substrat, comprenant les étapes :
(1) d'application d'une couche de recouvrement constituée d'une préparation selon l'une quelconque des revendications précédentes sur un substrat, et
(2) de décomposition thermique de la couche de recouvrement pour former une couche de platine.

10. Procédé selon la revendication 9, dans lequel le substrat est un ou plusieurs matériaux choisis dans le groupe constitué par le verre, la céramique, des substrats semiconducteurs, du métal, du plastique, des polymères modifiés ou non modifiés d'origine naturelle, des substrats de carbone, du carton et du papier.

11. Procédé selon la revendication 9 ou 10, dans lequel le substrat est pourvu de la couche de recouvrement sur la surface interne et/ou externe ou sur des parties de surface internes et/ou externes.

12. Procédé selon l'une des revendications 9 à 11, dans lequel la méthode d'application utilisée pour la réalisation de la couche de recouvrement est choisie dans le groupe constitué par le trempage, la pulvérisation, l'impression, l'application au pinceau, l'application à la brosse, l'application au feutre et l'application au chiffon.

13. Procédé selon l'une des revendications 9 à 12, dans lequel la couche de recouvrement appliquée à l'étape (1) est d'abord séchée, puis partiellement ou totalement libérée de solvant organique, avant d'être soumise à la décomposition thermique à l'étape (2).

14. Procédé selon l'une des revendications 9 à 13, dans lequel la décomposition thermique selon l'étape (2) est effectuée par traitement thermique, lequel traitement comprend un chauffage à une température d'objet supérieure à la température de décomposition de l'au moins un complexe de platine.

15. Procédé selon la revendication 14, dans lequel le chauffage à la température d'objet est effectué dans un four et/ou par rayonnement infrarouge.

16. Procédé selon l'une des revendications 9 à 15, dans lequel la couche de platine présente une épaisseur de 50 nm à 5 µm.
